# EUROPEAN PATENT APPLICATION

(11) **EP 4 703 351 A1**
(43) Date of publication of application: **04.03.2026**
(21) Application number: 24796399.4
(22) Date of filing: 24.04.2024
(51) Int. Cl.: C07C 209/36, C07C 211/57, C07C 201/12, C07C 205/06, C07C 205/56, C07D 471/04

(54) **METHOD OF PREPARING 4-AMINOINDAN AND METHOD OF PREPARING DIHYDROINDENYL-PYRAZOLO[3,4-B]PYRIDINE-AMINE COMPOUND**

(30) Priority: 25.04.2023 KR 20230054357
(71) Applicant: JW Pharmaceutical Corporation, Gyeonggi-do 13840 (KR)
(72) Inventor: LEE, Sumin, Seoul 06586 (KR); YOON, Dooha, Seongnam-si Gyeonggi-do 13562 (KR); CHIN, Seiho, Hwaseong-si Gyeonggi-do 18297 (KR)
(74) Representative: HGF
(86) International application number: PCT/IB2024/053975
(87) International publication number: WO 2024/224298

(57) **Abstract**

The present invention relates to a method of preparing 4-aminoindan and a method of preparing a dihydroindenyl-pyrazolo[3,4-b]pyridine-amine compound, wherein the method of preparing 4-aminoindan comprises the steps of: (Sa) using compound 1 to prepare compound 2; and (Sb) using compound 2 to prepare 4-aminoindan.

## Description

### TECHNICAL FIELD

The present invention relates to a method for preparing 4-aminoindan and a method for preparing a dihydroindenyl-pyrazolo[3,4-b]pyridine amine.

### BACKGROUND ART

4-aminoindan is one of the most widely used compounds in the medical and chemical arts per se or as a reactant for the preparation of various compounds. An IUPAC name of 4-aminoindan is 2,3-dihydro-1H-inden-4-amine.

As a method for synthesizing 4-aminoindan, there is disclosed a method for performing a nitration reaction using indan as a starting material to obtain 4-nitroindan, and reducing the same. However, in the nitration reaction according to reaction formula A below (under HNO₃/H₂SO₄ condition), the selectivity for position 5 of indan is higher than that of position 4, and thus 5-nitroindan is rather obtained as a main product, thereby making it difficult to separate and purify 4-nitroindan. In other words, such method has a problem in that 4-aminoindan is hardly mass-produced at a high yield.

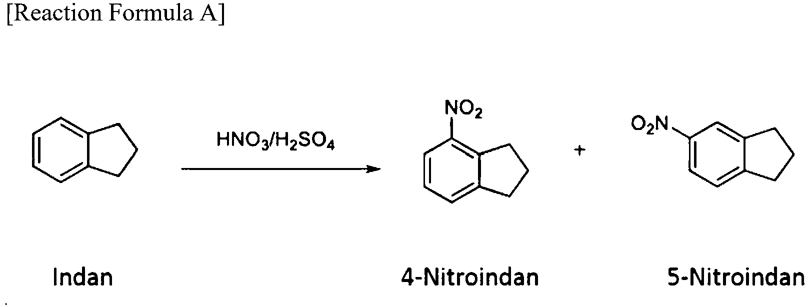

### [Related Art Reference]

### [Patent Documents]

(Patent Document 1) International Publication WO 2019/211463
(Patent Document 2) International Publication WO 2019/212256

### DETAILED DESCRIPTION OF THE INVENTION

### TECHNICAL PROBLEM

One object of the present invention is to provide a method for preparing 4-aminoindan capable of selectively mass-producing only 4-aminoindan at a high yield.

One object of the present invention is to provide a method for preparing a dihydroindenyl-pyrazolo[3,4-b]pyridine amine.

### TECHNICAL SOLUTION

A method for preparing 4-aminoindan for one object of the present invention includes:
(Sa) a step of reacting compound 1 represented by chemical formula 1 to prepare compound 2 represented by chemical formula 2; and
(Sb) a step of reacting the compound 2 to prepare 4-aminoindan represented by chemical formula 3.

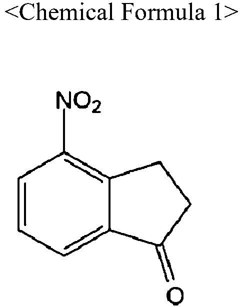

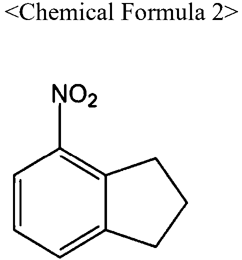

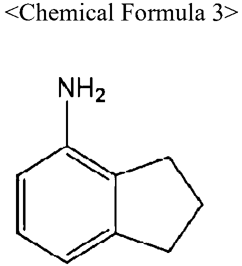

In one example, the step (Sa) may include a step of reacting the compound 1 with triethylsilane.

In one example, the step (Sa) may include a step of reacting compound 4 represented by chemical formula 4 to prepare the compound 1.

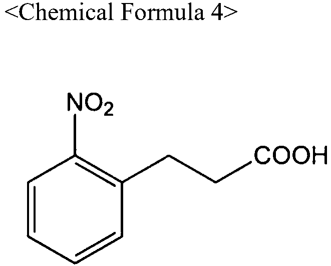

In one example, the step of preparing compound 1 may include a step of reacting the compound 4 with trifluoromethanesulfonic acid (TfOH).

In one example, the step (Sb) may include a step of performing a catalytic (catalyst) hydrogenation reaction.

In one example, the catalyst may be Pd/C.

In one example, the step (Sa) may include a step of reacting a compound 5 represented by chemical formula 5 to prepare the compound 4.

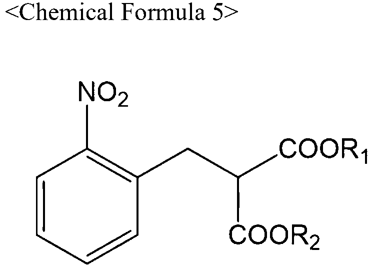

In chemical formula 5, R₁ and R₂ are each independently linear or branched C1-C6 alkyl, C3-C10 cycloalkyl, C6-C12 aryl, 4- to 7-membered heterocycloalkyl, or 5- to 7-membered heteroaryl.

In this case, the "alkyl" may be methyl, ethyl, propyl, isopropyl, etc., the "cycloalkyl" may be cyclobutyl, cyclopentyl, etc., and the "aryl" may be phenyl, biphenyl, naphthyl, etc. The "heterocycloalkyl" may be oxetanyl, piperidinyl, etc., and the "heteroaryl" may be pyridinyl, pyrimidinyl, etc.

In one example, the step (Sa) may include a step of reacting a compound 6 represented by chemical formula 6 to prepare the compound 5.

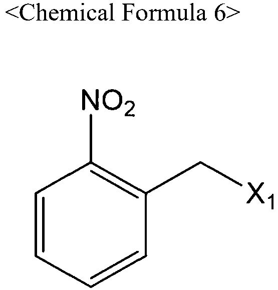

In chemical formula 6, X₁ is F, Cl, Br or I.

In one example, the step of preparing the compound 5 may include a step of reacting the compound 6 with a dialkyl malonate represented by chemical formula 7.

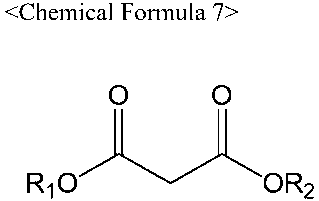

In the chemical formula 7, R₁ and R₂ are each independently linear or branched C1-C6 alkyl, C3-C10 cycloalkyl, C6-C12 aryl, 4- to 7-membered heterocycloalkyl, or 5- to 7-membered heteroaryl.

In one example, the step of preparing the compound 5 may include a step of reacting the compound 6 with lithium hydroxide monohydrate, tetrabutylammonium iodide (TBAI) and acetonitrile.

Each of the steps may exhibit a preparation yield of at least 70% or more, for example, 70% or more, 60% or more, 50% or more, 40% or more, 30% or more, or 20% or more.

In one example, the step (Sa) may include:
(S1) a step of preparing a compound 5 using a compound 6;
(S2) a step of preparing compound 4 using the compound 5; and
(S3) a step of preparing compound 1 using compound 4.

In this case, the steps (S1) to (S3) are substantially the same as those described in the step (Sa), and a redundant detailed description will be omitted.

A method for preparing 4-aminoindan for one object of the present invention may include:
a step of preparing a compound 5 using a compound 6;
a step of preparing compound 4 using the compound 5;
a step of preparing compound 1 using compound 4;
a step of preparing compound 2 using compound 1; and
a step of preparing 4-aminoindan using compound 2.

In this case, each step is substantially the same as those described in the steps (Sa) and (Sb), and a redundant detailed description will be omitted.

In the method for preparing a dihydroindenyl-pyrazolo[3,4-b]pyridine amine according to the present invention, the dihydroindenyl-pyrazolo[3,4-b]pyridine amine is a compound represented by chemical formula X below.

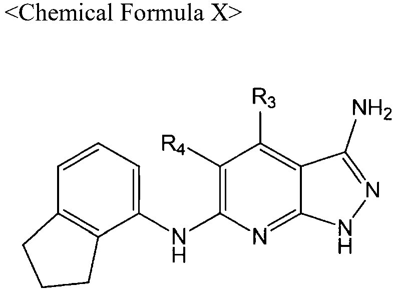

In chemical formula X, R₃ and R₄ are each independently H, OH, C1-C6 alkyl, C1-C6 haloalkyl, halogen, COOH, COO(C1-C6 alkyl), or C6-C12 aryl.

The definition of each of alkyl and aryl may be substantially the same as that described in R₁ and R₂, "haloalkyl" may be CF₃, CF₂H, CH₂CF₃, etc., and may be a functional group in which at least one of H of alkyl is substituted with halogen, and "halogen" may be F, Cl, Br, or I.

The method for preparing a dihydroindenyl-pyrazolo[3,4-b]pyridine amine according to the present invention may include:
a step of preparing compound 2 using compound 1;
a step of preparing 4-aminoindan using compound 2;
a step of reacting 4-aminoindan with a compound represented by chemical formula 8 to prepare a compound represented by chemical formula 9; and
a step of performing a cyclization reaction on the compound represented by chemical formula 9 to prepare a dihydroindenyl-pyrazolo[3,4-b]pyridine amine represented by chemical formula X.

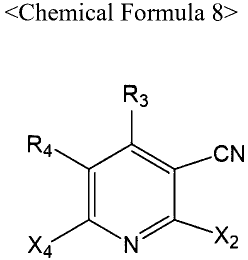

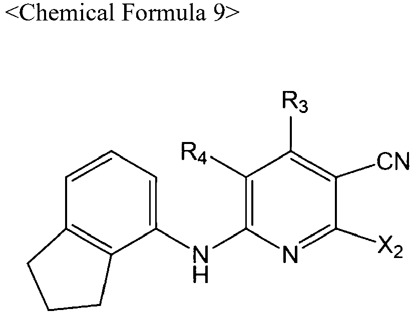

In chemical formulas 8 and 9, R₃ and R₄ are the same as defined in chemical formula X.

In chemical formulas 8 and 9, X₂ is Cl or Br.

The cyclization reaction may be carried out by reacting the compound represented by chemical formula 9 with hydrazine monohydrate.

In one example, a method for preparing compound 1 may not be particularly limited, but may be prepared by using compound 4 as a starting material as described above, and in this case, compound 4 may be prepared by using a compound 5. In addition, the compound 5 may be prepared by using a compound 6 as a starting material. A redundant detailed description will be omitted.

In one example, the present invention may provide a method for preparing N6-(2,3-dihydro-1H-inden-4-yl)-5-fluoro-1H-pyrazolo[3,4-b]pyridin-3,6-diamine.

A method for preparing N6-(2,3-dihydro-1H-inden-4-yl)-5-fluoro-1H-pyrazolo[3,4-b]pyridin-3,6-diamine may include:
a step of preparing compound 2 using compound 1;
a step of preparing 4-aminoindan using compound 2;
a step of reacting 4-aminoindan with 2,6-dichloro-5-fluoronicotinonitrile to prepare 2-chloro-6-((2,3-dihydro-1H-inden-4-yl)amino)-5-fluoronicotinonitrile; and
a step of performing a cyclization reaction on 2-chloro-6-((2,3-dihydro-1H-inden-4-yl)amino)-5-fluoronicotinonitrile to prepare N6-(2,3-dihydro-1H-inden-4-yl)-5-fluoro-1H-pyrazolo[3,4-b]pyridine-3,6-diamine.

In one example, the step of preparing 2-chloro-6-((2,3-dihydro-1H-inden-4-yl)amino)-5-fluoronicotinonitrile may use dimethyl sulfoxide (DMSO) and N,N-diisopropylethylamine (DIPEA) when reacting 4-aminoindan with 2,6-dichloro-5-fluoronicotinonitrile. The reaction under the solvent condition may easily obtain 2-chloro-6-((2,3-dihydro-1H-inden-4-yl)amino)-5-fluoronicotinonitrile without using a harmful substance such as NMP or 2-methoxyethanol.

In this case, compound 1 may be prepared through a step of preparing a compound 5 using a compound 6; a step of preparing compound 4 using the compound 5; and a step of preparing compound 1 using compound 4. A redundant detailed description will be omitted.

According to the method for preparing the dihydroindenyl-pyrazolo[3,4-b]pyridine amine of the present invention, 4-aminoindan may be mass-produced at a high yield, and thus a preparation yield of the dihydroindenyl-pyrazolo[3,4-b]pyridine amine using 4-aminoindan as an essential reactant may also be at least 70% or higher, for example, 70% or higher, 60% or higher, 50% or higher, 40% or higher, 30% or higher, or 20% or higher, which may be remarkably improved compared to the related art. In addition, the dihydroindenyl-pyrazolo[3,4-b]pyridine amine may be obtained at a high yield.
1) The present invention provides a method for preparing 4-aminoindan, the method including: (Sa) a step of reacting compound 1 represented by chemical formula 1 to prepare compound 2 represented by chemical formula 2; and (Sb) a step of reacting the compound 2 to prepare 4-aminoindan represented by chemical formula 3: 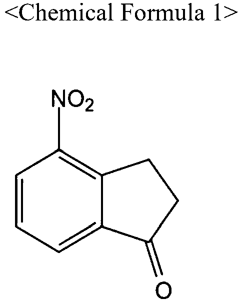 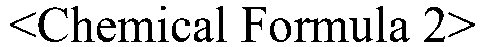 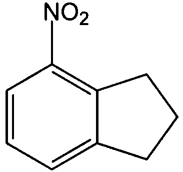 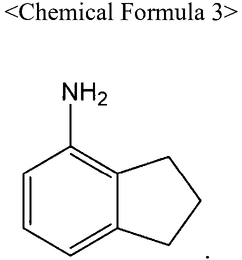
2) In 1), the step (Sa) may include a step of reacting the compound 1 with triethylsilane.
3) In 1) or 2), the step (Sa) may include a step of reacting compound 4 represented by chemical formula 4 to prepare the compound 1: 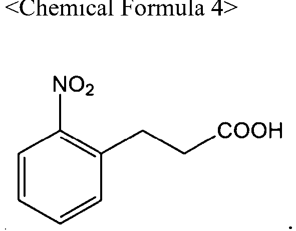
4) In 3), the step of preparing compound 1 may include a step of reacting the compound 4 with trifluoromethanesulfonic acid (TfOH).
5) In 3) or 4), the step (Sa) may include a step of reacting a compound 5 represented by chemical formula 5 to prepare the compound 4:

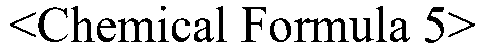

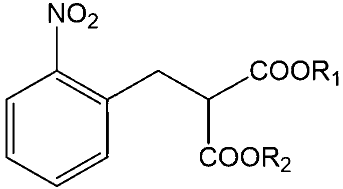

In chemical formula 5, R₁ and R₂ are each independently linear or branched C1-C6 alkyl, C3-C10 cycloalkyl, C6-C12 aryl, 4- to 7-membered heterocycloalkyl, or 5- to 7-membered heteroaryl.
6) In 5), the step (Sa) may include a step of reacting a compound 6 represented by chemical formula 6 to prepare the compound 5.
7) In 6), the step of preparing compound 5 may include a step of reacting the compound 6 with a dialkyl malonate.
8) In 6) or 7), the step of preparing the compound 5 may include a step of reacting the compound 6 with lithium hydroxide monohydrate, tetrabutylammonium iodide (TBAI) and acetonitrile.
9) In any one of 1) to 8), the step (Sb) may include a step of performing a catalytic (catalyst) hydrogenation reaction.
10) The present invention may provide a method for preparing 4-aminoindan, the method including: a step of reacting a compound 6 represented by chemical formula 6 to prepare a compound 5 represented by chemical formula 5; a step of reacting the compound 5 to prepare compound 4 represented by chemical formula 4; a step of reacting compound 4 to prepare compound 1 represented by chemical formula 1; a step of reacting compound 1 to prepare compound 2 represented by chemical formula 2; and a step of reacting compound 2 to prepare 4-aminoindan:

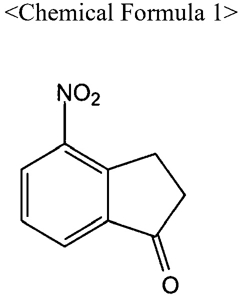

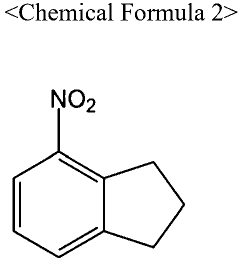

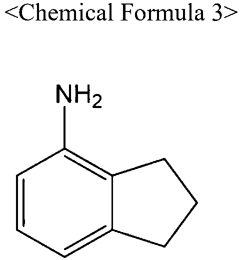

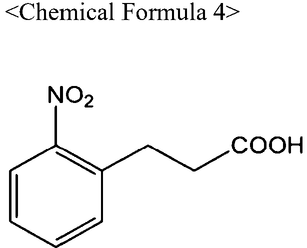

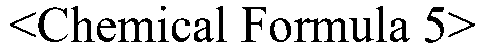

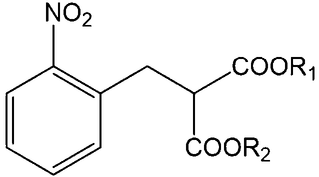

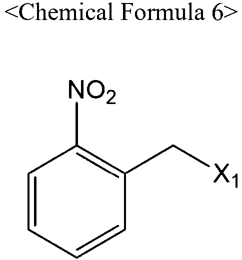

In chemical formula 5, R₁ and R₂ are each independently linear or branched C1-C6 alkyl, C3-C10 cycloalkyl, C6-C12 aryl, 4- to 7-membered heterocycloalkyl, or 5- to 7-membered heteroaryl. In chemical formula 6, X₁ is F, Cl, Br or I.
11) In 10), the step of preparing compound 1 may include a step of reacting the compound 4 with trifluoromethanesulfonic acid (TfOH).
12) In 10) or 11), the step of preparing compound 2 may include a step of reacting the compound 1 with triethylsilane.
13) In any one of 10) to 12), the step of preparing a compound 5 may include reacting the compound 6 with dialkyl malonate.
14) In any one of 10) to 13), the step of preparing the compound 5 may include a step of reacting the compound 6 with a salt of lithium hydroxide monohydrate, tetrabutylammonium iodide (TBAI) and acetonitrile.
15) The present invention provides a method for preparing a dihydroindenyl-pyrazolo[3,4-b]pyridine amine, the method including: a step of reacting compound 1 represented by chemical formula 1 to prepare compound 2 represented by chemical formula 2; a step of reacting compound 2 to prepare 4-aminoindan represented by chemical formula 3; a step of reacting 4-aminoindan with a compound represented by chemical formula 8 to prepare a compound represented by chemical formula 9; and a step of performing a cyclization reaction on the compound represented by chemical formula 9 to prepare a dihydroindenyl-pyrazolo[3,4-b]pyridine amine represented by chemical formula X:

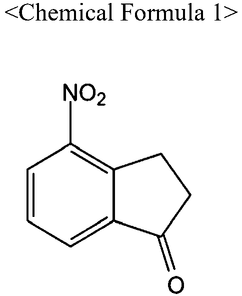

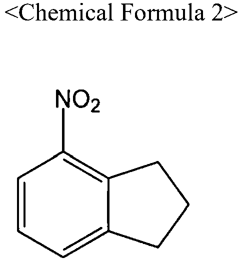

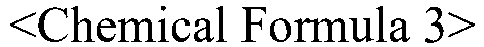

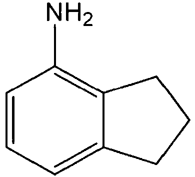

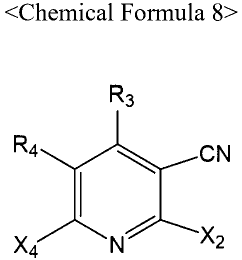

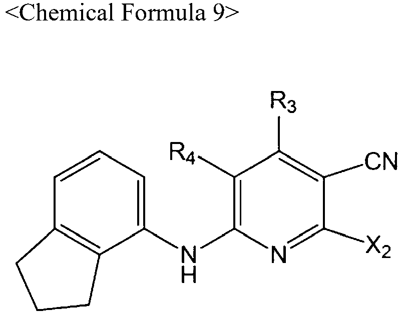

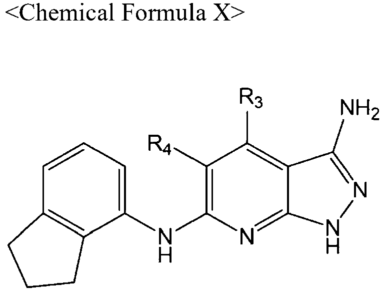

In chemical formulas 8, 9 and X, R₃ and R₄ are each independently H, OH, C1-C6 alkyl, C1-C6 haloalkyl, halogen, COOH, COO(C1-C6 alkyl), or C6-C12 aryl, and
in chemical formulas 8 and 9, X₂ is Cl or Br.
16) In 15), the dihydroindenyl-pyrazolo[3,4-b]pyridine amine may be N6-(2,3-dihydro-1H-inden-4-yl)-5-fluoro-1H-pyrazolo[3,4-b]pyridin-3,6-diamine.

### ADVANTAGEOUS EFFECTS

According to the method for preparing 4-aminoindan and the method for preparing the dihydroindenyl-pyrazolo[3,4-b]pyridine amine of the present invention described above, 5-aminoindan cannot be produced at all during the preparation process, and thus the process of separately separating 4-aminoindan can be fundamentally omitted. In addition, each step can exhibit a preparation yield of at least 70% or more, and thus an overall preparation yield of 4-aminoindan can be remarkably improved. Accordingly, 4-aminoindan can be mass-produced at a high yield, and the dihydroindenyl-pyrazolo[3,4-b]pyridine amine can also be prepared at a high yield and with a high purity.

### BRIEF DESCRIPTION OF THE DRAWINGS

FIG. 1 is a view showing an HPLC analysis graph of a product obtained in step 5 of Example 1 of the present invention.
FIG. 2 is a view showing an HPLC analysis graph of a product obtained in step 4 of Example 1 of the present invention.

### MODE FOR INVENTION

Terms used in the present application are used only to describe a certain example and are not intended to limit the present invention. In the present application, terms such as "include," "have" or the like shall be intended to designate a presence of features, steps, operations, components, or combinations thereof described herein, and shall not be construed to exclude a possible presence or addition of one or more other features, steps, operations, components, or combinations thereof in advance. All the terms used herein including technical or scientific terms have the same meaning as commonly understood by those ordinary skilled in the art, to which the present invention pertains, unless defined otherwise.

### Example 1: Method for preparing 4-aminoindan

### (Step 1) Preparation of dimethyl 2-(2-nitrobenzyl)malonate (compound 5)

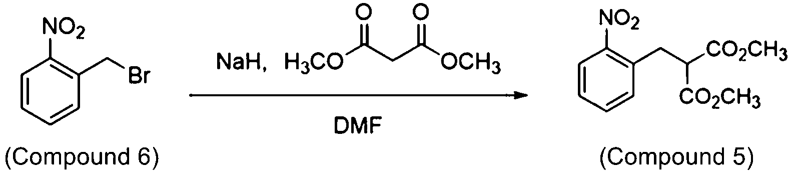

Dimethylformamide (DMF) and NaH (60%) (1.6 eq.) were added into a reactor filled with nitrogen at room temperature (20 to 30°C), and a temperature was adjusted to 0 to 10°C. In this case, a weight (unit: g) of compound 6 to be used as a starting material was determined to be 1 eq., and then an amount of the DMF added was a volume (mL) of 3.7 times the weight (g) corresponding to 1 eq. of compound 6. Then, dimethyl malonate was slowly added to the reaction solution and stirred at 20 to 30°C for one hour. Compound 6 (1 eq.) was dissolved in the DMF (volume (mL) twice the weight (g) corresponding to 1 eq. of compound 6), slowly added to the reaction solution, and stirred at 20 to 30°C for one hour. Water (volume (mL) of three times the weight (g) corresponding to 1 eq. of compound 6) and ethyl acetate (volume (mL) of three times the weight (g) corresponding to 1 eq. of compound 6) were added thereto and stirred for ten minutes. An organic phase was separated and washed with NH₄Cl solution. The organic phase was separated again, and dehydrated and filtered with magnesium sulfate (MgSO₄). A filtered filtrate was concentrated to obtain the title compound 5.

In addition, dimethyl 2-(2-nitrobenzyl)malonate (compound 5) may be prepared by the following method.

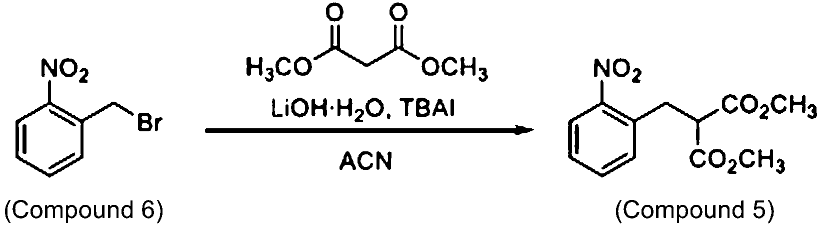

Compound 6 (1 eq.), diethyl malonate (2 eq. of compound 6), lithium hydroxide monohydrate (2 eq. of compound 6), TBAI (0.05 eq. of compound 6), and acetonitrile were added to a reactor filled with nitrogen at room temperature (20 to 30°C) and then stirred at a temperature of 20 to 30°C for 1.5 hours. In this case, an amount of acetonitrile (ACN) solvent added was a volume (ml) of 20 times the weight (g) corresponding to 1 eq. of compound 6.

10% HCl (volume (ml) of 20 times the weight (g) corresponding to 1 eq. of compound 6) and ethyl acetate (volume (ml) of 20 times the weight (g) corresponding to 1 eq. of compound 6) were added thereto and stirred for ten minutes. An organic phase was separated and washed with water (volume (ml) of 20 times the weight (g) corresponding to 1 eq. of compound 6). The organic phase was separated again, and dehydrated and filtered with magnesium sulfate. A filtered filtrate was concentrated to obtain the title compound 5.
¹H NMR : 7.977-7.960 (1H, m), 7.679-7.646 (1H, m), 7.526-7.491 (2H, m), 3.932 (1H, t, J=7.82 Hz), 3.613 (6H, s), 3.401 (2H, m)
¹³C NMR : 168.474, 133.423, 132.286, 132.004, 128.456, 124.687, 52.503, 51.695, 30.614.

### (Step 2) Preparation of compound 4

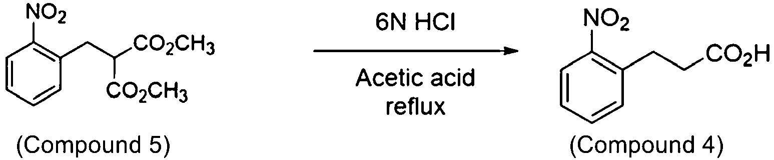

Compound 5 (1 eq.) obtained according to the process of the step 1, 6 N HCl (volume (mL) of 6.1 times the weight (g) corresponding to 1 eq. of compound 5) and acetic acid (volume (mL) of 6.1 times the weight (g) corresponding to 1 eq. of compound 5) were added into a reactor filled with nitrogen. A weight of compound 5 used was directly obtained in the step 1. A temperature was raised to 100°C and stirred for five hours, and the reaction solution was concentrated. Water (volume (mL) of ten times the weight (g) corresponding to 1 eq. of compound 5) was added into the concentrated residue and the pH of the reaction solution was adjusted to 12 with 10% NaOH. Subsequently, ethyl acetate (volume (mL) of five times the weight (g) corresponding to 1 eq. of compound 5) was added and stirred for ten minutes, and an aqueous phase was separated. For the separated aqueous phase, the pH was adjusted to 1 with 5% HCl. Ethyl acetate (volume (mL) of five times the weight (g) corresponding to 1 eq. of compound 5) was added to the reaction solution and stirred for ten minutes. An organic phase was separated, dehydrated and filtered with magnesium sulfate, and the filtrate was concentrated and purified by column chromatography with ethyl acetate and hexane to obtain the title compound 4 (light brown solid, overall yield 81% of steps 1 and 2).
¹H NMR : 12.248 (1H, brs), 7.915 (1H, m), 7.643 (1H, m), 7.526 (1H, m), 7.448 (1H, m), 3.020 (2H, t, *J*=7.57 Hz), 2.572 (2H, t, *J*=7.57 Hz)
¹³C NMR : 173.52, 149.38, 135.31, 133.51, 131.95, 127.88, 124.55, 34.44, 27.36.

### (Step 3) Preparation of compound 1

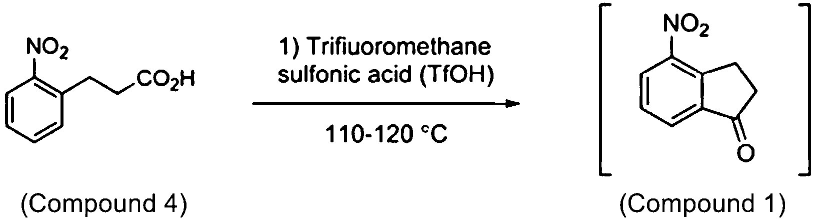

Compound 4 (1 eq.) obtained according to the process of the step 2 and trifluoromethanesulfonic acid (volume (mL) of ten times the weight (g) corresponding to 1 eq. of compound 4) were added to a reactor filled with nitrogen. A temperature was raised to 110 to 120°C, stirred for 2.5 hours, and cooled to 25°C to obtain a reaction solution containing the title compound 1 (dark brown solid, yield 74.25%).
¹H NMR : 8.495 (1H, d, J=8.30 Hz), 8.058 (1H, d, J=7.33 Hz), 7.734 (1H, t, d=7.82 Hz), 3.531-3.509 (2H, m), 2.755-2.731 (2H, m)
¹³C NMR : 204.63, 149.80, 146.08, 139.71, 129.90, 129.27, 129.20, 35.59, 26.49.

### (Step 4) Preparation of compound 2

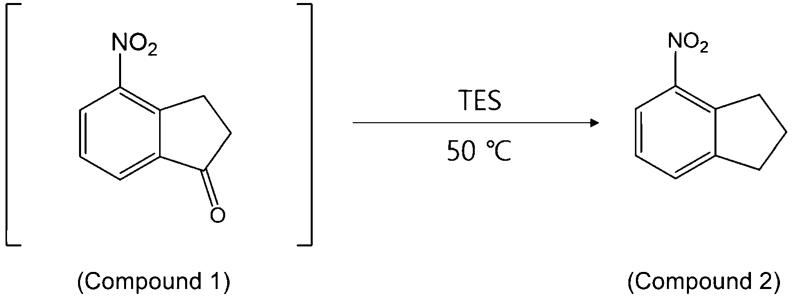

Triethylsilane (13 eq.) was added into the reaction solution obtained in the step 3, and a temperature was raised to 50°C and stirred for 12 hours. After cooling to 25°C, water (volume (mL) of ten times the weight (g) corresponding to 1 eq. of compound 1) and ethyl acetate (volume (mL) of ten times the weight (g) corresponding to 1 eq. of compound 1) were added to the reaction solution and stirred for ten minutes. An organic phase was separated and washed with NaHCO₃ solution. The organic phase was separated again and washed with water, and then the organic phase was separated again, and dehydrated and filtered with magnesium sulfate. A filtrate was concentrated and purified by column chromatography using 10% ethyl acetate to obtain the title compound 2 (pale yellow solid, yield 85.41%).
¹H NMR : 8.486-8.469 (1H, m), 8.048-8.034 (1H, m), 7.732-7.701 (1H, m), 3.514-3.491 (2H, m), 2.737-2.714 (2H, m)
¹³C NMR : 147.94, 145.19, 139.88, 130.40, 127.68, 121.54, 33.31, 24.28.

### (Step 5) Preparation of 4-aminoindan (compound 3)

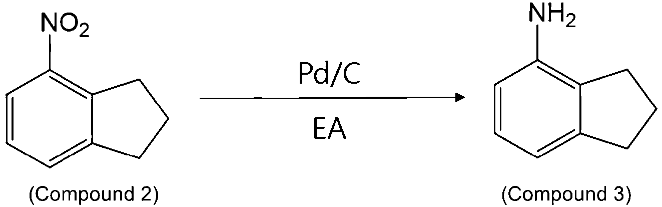

Compound 2 (1 eq.) obtained according to the process of the step 4 and ethyl acetate were added to a reactor filled with nitrogen at room temperature (20 to 30°C). Subsequently, 10% Pd/C (20 wt%) was added into the mixed solution, and a balloon containing H2 gas was added into the reaction solution to inject H2 gas. The resulting mixture was stirred at 24 to 30°C for two hours, filtered to remove impurities, and then the filtrate was concentrated to obtain the title compound 3 (brown oil, yield 81.22%).
¹H NMR : 7.026-6.996 (1H, m), 6.727-6.713 (1H, m), 6.527-6.511 (1H, m), 2.935 (2H, t, J=7.33 Hz), 2.750 (2H, t, J=7.33 Hz), 2.152-2.092 (2H, m)
¹³C NMR : 144.29, 144.02, 127.09, 126.83, 112.10, 111.37, 32.80, 29.62, 24.23

### Comparative Example 1

Step A and step B of "Intermediate R4" of International Publication WO2019/211463 were sequentially performed to prepare 4-aminoindan.

A yield of the product (nitroindan) obtained according to step A of "Intermediate R4" was 53.51%, and a yield of the product (aminoindan) obtained according to step B of "Intermediate R4" was 42.55%.

As a result of analyzing the product (nitroindan) obtained according to step A of "Intermediate R4" via HPLC, it was confirmed that 4-nitroindan and 5-nitroindan are mixed at a ratio of 35 : 75 to 40 : 60. When 4-aminoindan and 5-aminoindan were mixed at the ratio range even in the product (aminoindan) obtained by directly applying the product (nitroindan) obtained according to step A of "Intermediate R4" to step B, and 4-aminoindan was obtained from the product obtained according to step B, that is, a preparation yield of only 4-aminoindan excluding 5-aminoindan from the product obtained through step A and step B using indan as a starting material was 7.9 to 9.1%.

### Evaluation 1 - Confirmation of inclusion of 5-aminoindan

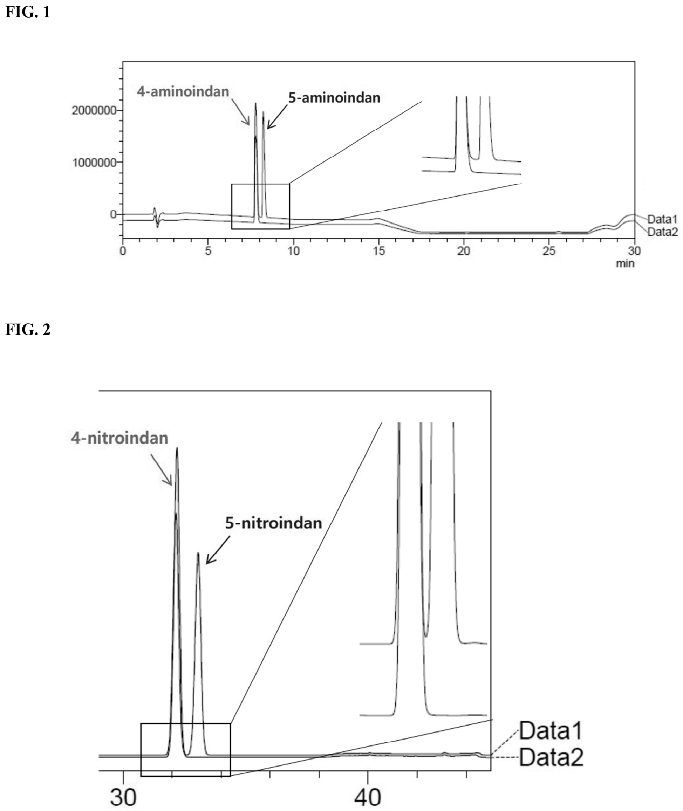

An HPLC analysis was performed on the product obtained in step 5 of Example 1 of the present invention. As a control sample, a mixture of 4-aminoindan and 5-aminoindan was used. The results of the HPLC analysis are shown in FIG. 1.

Referring to FIG. 1, it can be confirmed that each peak of 4-aminoindan and 5-aminoindan appears in the mixture of 4-aminoindan and 5-aminoindan (see a graph of data 1). On the contrary, in the product obtained in step 5 of Example 1 of the present invention, it can be confirmed that only one peak appears (see a graph of DATA 2), and as a result of comparing the same with the peaks of the control sample, a corresponding peak may be a peak for 4-aminoindan. In other words, it can be confirmed that only 4-aminoindan may be selectively prepared through steps 1 to 5 of the present invention.

Accordingly, with regard to the product obtained in step 5 of Example 1 of the present invention, it can be confirmed that only 4-aminoindan is selectively obtained at 100% even without a separate purification process in step 5.

### Evaluation 2 - Confirmation of inclusion of 5-nitroindan

An HPLC analysis was performed on the product obtained in step 4 of Example 1 of the present invention. As a control sample, a mixture of 4-nitroindan and 5-nitroindan was used. The results of the HPLC analysis are shown in FIG. 2.

Referring to FIG. 2, it can be confirmed that each peak of 4-nitroindan and 5-nitroindan appears in the mixture of 4-nitroindan and 5-nitroindan (see a graph of DATA 1). On the contrary, in the product obtained in step 4 of Example 1 of the present invention, it can be confirmed that only one peak appears (see a graph of data 2), and as a result of comparing the same with the peaks of the control sample, a corresponding peak may be a peak for 4-nitroindan. In other words, it can be confirmed that only 4-nitroindan may be selectively prepared through steps 1 to 4 of the present invention.

### Example 2: Preparation of N6-(2,3-dihydro-1H-inden-4-yl)-5-fluoro-1H-pyrazolo[3,4-b]pyridin-3,6-diamine

### Steps 1 to 5: Preparation of 4-aminoindan

4-aminoindan was prepared according to substantially the same process as described in steps 1 to 5 of Example 1.

### Step 6: Preparation of 2-chloro-6-((2,3-dihydro-1H-inden-4-yl)amino)-5-fluoronicotinonitrile

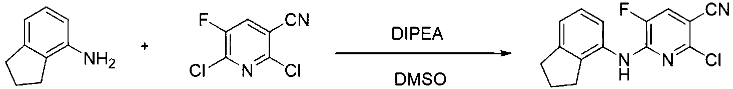

4-aminoindan obtained in the step 5, 2,6-dichloro-5-fluoronicotinonitrile, and dimethyl sulfoxide (DMSO) were added into a reactor dried and filled with nitrogen at room temperature (20 to 30°C), and then N,N-diisopropylethylamine (DIPEA) was added thereto. In this case, an amount of 2,6-dichloro-5-fluoronicotinonitrile added was 1.58 times the weight (kg), an amount of DMSO added was 11 times the weight (kg), and an amount of DIPEA added was 1.18 times the weight (kg) based on the weight (kg) of 4-aminoindan.

A temperature inside the reactor was raised to 100±5°C and stirred for three hours, and then a temperature inside the reactor was cooled to 25±5°C. When a temperature inside the reactor reached 25±5°C, distilled water was directly introduced without temperature control. In this case, the distilled water used was ten times the weight (kg) based on the weight (kg) of initially added 4-aminoindan. After the distilled water was completely added, a temperature inside the reactor was cooled to 25±5°C and stirred for 15 minutes. An obtained crystallized liquid was filtered and washed with distilled water (20 times the weight (kg) of an initial input weight (kg) of 4-aminoindan). An obtained filtered cake was dried under reduced pressure while an external temperature was set to 40°C or less. Accordingly, 2-chloro-6-((2,3-dihydro-1H-inden-4-yl)amino)-5-fluoronicotinonitrile (hereinafter, referred to as crude-WA) was obtained as crude.

Subsequently, dichloromethane (DCM) and the crude-WA obtained in the step were added into a reactor dried and filled with nitrogen at room temperature (20 to 30°C). At this time, an amount of DMC added was 13.3 times the weight (kg) based on the weight (kg) of the crude-WA.

A temperature inside the reactor was raised to 40±5°C, and it was confirmed whether or not all of the crude-WA in the reactor was dissolved. The resulting mixture was further stirred at the same temperature for 15 minutes. After that, the temperature inside the reactor was cooled to 25±5°C, and heptane was added when crystals were precipitated. An amount of heptane added was 20.52 times the weight (kg) based on the weight (kg) of the crude-WA added into the reactor. After the heptane was completely added, a temperature inside the reactor was cooled to 0±5°C. After the cooling was completed, the resulting product was cooled and aged at the same temperature for one hour, and the obtained crystallized liquid was filtered and washed with heptane. At this time, an amount of heptane used was 10.26 times the weight (kg) based on the weight (kg) of the crude-WA initially added into the reactor. An obtained filtered cake was dried under reduced pressure while an external temperature was set to 40°C or less. Accordingly, 2-chloro-6-((2,3-dihydro-1H-inden-4-yl)amino)-5-fluoronicotinonitrile was obtained (100 g of 4-aminoindan used in step 6, 181.73 g of 2-chloro-6-((2,3-dihydro-1H-inden-4-yl)amino)-5-fluoronicotinonitrile obtained, yield 88.25%, purity 99.6%).

### Step 7: Preparation of N6-(2,3-dihydro-1H-inden-4-yl)-5-fluoro-1H-pyrazolo[3,4-b]pyridin-3,6-diamine

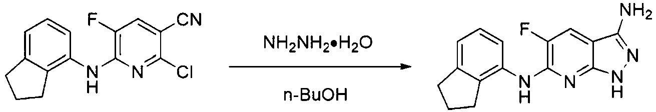

2-chloro-6-((2,3-dihydro-1H-inden-4-yl)amino)-5-fluoronicotinonitrile obtained in the step 6 and n-BuOH were added into a reactor dried and filled with nitrogen, and then hydrazine monohydrate was added thereto. In this case, an amount of n-BuOH added was 4.05 times the weight (kg) and an amount of hydrazine monohydrate was 1.16 times the weight (kg) based on the weight (kg) of 2-chloro-6-((2,3-dihydro-1H-inden-4-yl)amino)-5-fluoronicotinonitrile.

After raising a temperature inside the reactor to 105±5°C, the resulting mixture was stirred for seven hours while maintaining the internal temperature. Subsequently, the inside of the reactor was cooled to 0±5°C and then further stirred for one hour. An obtained crystallized liquid was filtered and washed with distilled water (ten times the weight (kg) of 2-chloro-6-((2,3-dihydro-1H-inden-4-yl)amino)-5-fluoronicotinonitrile added into the reactor). A filtered cake was dried under reduced pressure while an external temperature was set to 40°C or less. Accordingly, N6-(2,3-dihydro-1H-inden-4-yl)-5-fluoro-1H-pyrazolo[3,4-b]pyridin-3,6-diamine (hereinafter referred to as crude-product) was obtained as crude.

Subsequently, the crude-product obtained in the step and the DMSO were added into a reactor dried and filled with nitrogen at room temperature (20 to 30°C). In this case, an amount of the DMSO added was 5.5 times the weight (kg) based on the weight (kg) of the crude-product. When it was confirmed that all crude-products inside the reactor were dissolved, distilled water was added dropwise while maintaining the internal temperature at 25±5°C. In this case, an amount of distilled water added was three times the weight (kg) based on the weight (kg) of the crude-product added into the reactor. After the distilled water was completely added, the resulting mixture was aged and stirred for one hour while maintaining a temperature inside the reactor to 25±5°C. An obtained crystallized liquid was filtered and washed with distilled water (10 times the weight (kg) based on the weight (kg) of the crude-product added into the reactor). An obtained filtered cake was dried under reduced pressure while an external temperature was set to 40°C or less. Accordingly, N6-(2,3-dihydro-1H-inden-4-yl)-5-fluoro-1H-pyrazolo[3,4-b]pyridin-3,6-diamine was prepared (139.64 g of N6-(2,3-dihydro-1H-inden-4-yl)-5-fluoro-1H-pyrazolo[3,4-b]pyridin-3,6-diamine was obtained by directly using the final product obtained in step 7, yield 82.73% and purity 99.7%).

¹H NMR : 11.426 (s, 1 H), 11.392-11.468 (m, 1 H), 8.315 (s, 1 H), 7.731 (s, 1 H), 7.678-7.761 (m, 1 H), 7.359 (d, J=7.816 Hz, 1 H), 7.112 (t, J=7.572 Hz, 1 H), 7.011 (d, J=7.328 Hz, 1 H), 6.978-7.046 (m, 1 H), 5.213 (s, 2 H), 2.891 (t, J=7.328 Hz, 2 H), 2.754 (t, J=7.328 Hz, 2 H), 1.967 (quin, J=7.328 Hz, 2 H).

### Evaluation 3 - Quality and yield

It was confirmed that the purity of N6-(2,3-dihydro-1H-inden-4-yl)-5-fluoro-1H-pyrazolo[3,4-b]pyridine-3,6-diamine, which is the final product obtained in Example 2, is 99.7%, which is very excellent. In particular, it was also confirmed that the purity obtained by the method for preparing N6-(2,3-dihydro-1H-inden-4-yl)-5-fluoro-1H-pyrazolo[3,4-b]pyridin-3,6-diamine according to the present invention is improved compared to the purity of N6-(2,3-dihydro-1H-inden-4-yl)-5-fluoro-1H-pyrazolo[3,4-b]pyridin-3,6-diamine prepared by preparing 4-aminoindan according to the conventional method and using the same.

In addition, since an overall preparation yield of the method for preparing N6-(2,3-dihydro-1H-inden-4-yl)-5-fluoro-1H-pyrazolo[3,4-b]pyridin-3,6-diamine according to the present invention uses the method for preparing the 4-aminoindan of the present invention with remarkably improved productivity compared to the conventional method, it can be confirmed that the overall preparation yield of the method according to the present invention is significantly improved at least four times or more compared to the preparation yield of the conventional method for preparing 4-aminoindan and using the same to prepare N6-(2,3-dihydro-1H-inden-4-yl)-5-fluoro-1H-pyrazolo[3,4-b]pyridin-3,6-diamine.

Even in a method for synthesizing N6-(2,3-dihydro-1H-inden-4-yl)-5-fluoro-1H-pyrazolo[3,4-b]pyridin-3,6-diamine using 4-aminoindan as a starting material in the same way, in the case of obtaining 4-aminoindan according to the present invention and preparing N6-(2,3-dihydro-1H-inden-4-yl)-5-fluoro-1H-pyrazolo[3,4-b]pyridin-3,6-diamine, it was confirmed that the preparation yield is excellent compared to the case of obtaining 4-aminoindan by the conventional method and preparing N6-(2,3-dihydro-1H-inden-4-yl)-5-fluoro-1H-pyrazolo[3,4-b]pyridin-3,6-diamine.

The present invention has been described with reference to preferred exemplary embodiments herein, but it will be understood by those skilled in the art that the present invention may be variously changed and modified without departing from the spirit and field of the present invention, as described in the following scope of patent claims.

## Claims

1. A method for preparing 4-aminoindan, the method comprising:
(Sa) a step of reacting compound 1 represented by chemical formula 1 to prepare compound 2 represented by chemical formula 2; and
(Sb) a step of reacting the compound 2 to prepare 4-aminoindan represented by chemical formula 3:

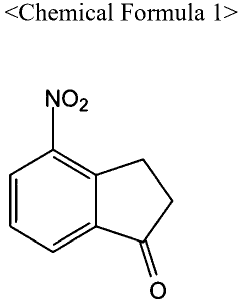

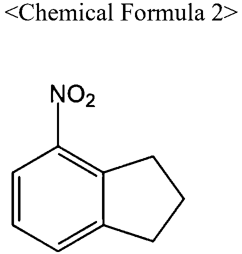

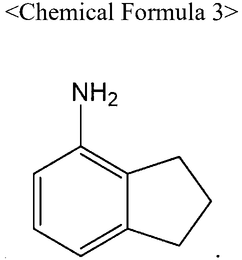

2. The method of claim 1, wherein the step (Sa) comprises a step of reacting the compound 1 with triethylsilane.

3. The method of claim 1, wherein the step (Sa) comprises a step of reacting compound 4 represented by chemical formula 4 to prepare the compound 1:

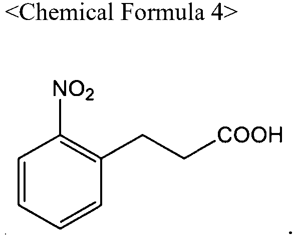

4. The method of claim 3, wherein the step of preparing compound 1 comprises a step of reacting the compound 4 with trifluoromethanesulfonic acid (TfOH).

5. The method of claim 3, wherein the step (Sa) comprises a step of reacting a compound 5 represented by chemical formula 5 to prepare the compound 4: 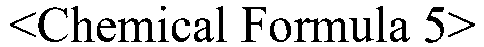 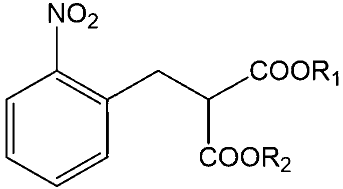 wherein in chemical formula 5, R₁ and R₂ are each independently linear or branched C1-C6 alkyl, C3-C10 cycloalkyl, C6-C12 aryl, 4- to 7-membered heterocycloalkyl, or 5- to 7-membered heteroaryl.

6. The method of claim 1, wherein the step (Sb) comprises a step of performing a catalytic (catalyst) hydrogenation reaction.

7. A method for preparing 4-aminoindan, the method comprising:
a step of reacting a compound 6 represented by chemical formula 6 to prepare a compound 5 represented by chemical formula 5;
a step of reacting the compound 5 to prepare compound 4 represented by chemical formula 4;
a step of reacting compound 4 to prepare compound 1 represented by chemical formula 1;
a step of reacting compound 1 to prepare compound 2 represented by chemical formula 2; and
a step of reacting compound 2 to prepare 4-aminoindan: 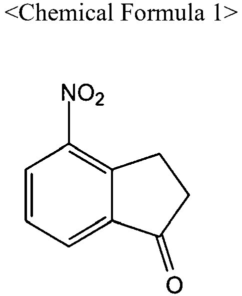 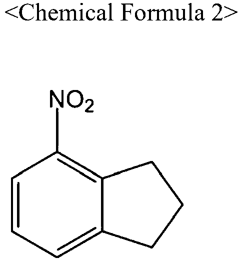 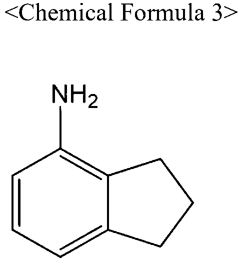 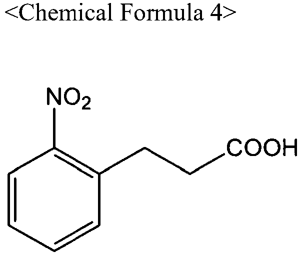 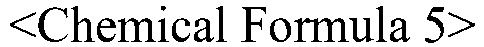 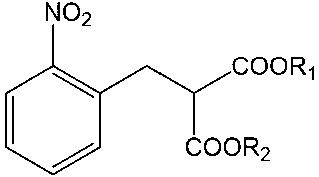 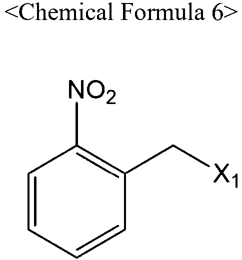
wherein in chemical formula 5, R₁ and R₂ are each independently linear or branched C1-C6 alkyl, C3-C10 cycloalkyl, C6-C12 aryl, 4- to 7-membered heterocycloalkyl, or 5- to 7-membered heteroaryl, and
wherein, in chemical formula 6, X₁ is F, Cl, Br or I.

8. The method of claim 7, wherein the step of preparing compound 1 comprises a step of reacting the compound 4 with trifluoromethanesulfonic acid (TfOH).

9. The method of claim 7, wherein the step of preparing compound 2 comprises a step of reacting the compound 1 with triethylsilane.

10. The method of claim 7, wherein the step of preparing the compound 5 comprises a step of reacting the compound 6 with dialkyl malonate.

11. The method of claim 7, wherein the step of preparing the compound 5 comprises a step of reacting the compound 6 with lithium hydroxide monohydrate, tetrabutylammonium iodide (TBAI) and acetonitrile.

12. A method for preparing a dihydroindenyl-pyrazolo[3,4-b]pyridine amine, the method comprising:
a step of reacting compound 1 represented by chemical formula 1 to prepare compound 2 represented by chemical formula 2;
a step of reacting compound 2 to prepare 4-aminoindan represented by chemical formula 3;
a step of reacting 4-aminoindan with a compound represented by chemical formula 8 to prepare a compound represented by chemical formula 9; and
a step of performing a cyclization reaction on the compound represented by chemical formula 9 to prepare a dihydroindenyl-pyrazolo[3,4-b]pyridine amine represented by chemical formula X: 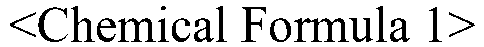 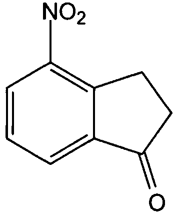 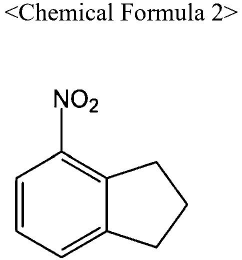 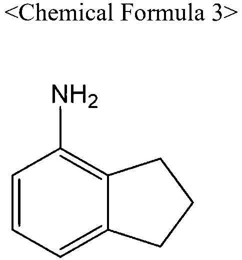 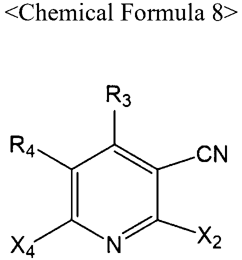 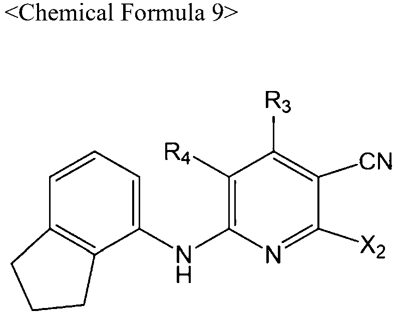 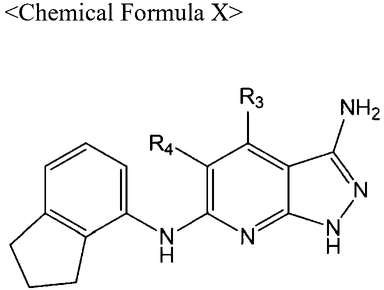
wherein in chemical formulas 8, 9 and X, R₃ and R₄ are each independently H, OH, C1-C6 alkyl, C1-C6 haloalkyl, halogen, COOH, COO(C1-C6 alkyl), or C6-C12 aryl, and
wherein in chemical formulas 8 and 9, X₂ is Cl or Br.

13. The method of claim 12, wherein the dihydroindenyl-pyrazolo[3,4-b]pyridine amine is N6-(2,3-dihydro-1H-inden-4-yl)-5-fluoro-1H-pyrazolo[3,4-b]pyridin-3,6-diamine.
